# EUROPEAN PATENT APPLICATION

(11) **EP 1 891 937 A1**
(43) Date of publication of application: **27.02.2008**
(21) Application number: 06119520.2
(22) Date of filing: 25.08.2006
(51) Int. Cl.: A61K 9/00, A61K 9/20, A61K 31/165

(54) **Galenic formulations of aliskiren**

(71) Applicant: Novartis AG, 4056 Basel (CH)
(72) Inventor: The designation of the inventor has not yet been filed
(74) Representative: Hillebrand, Dirk

(57) **Abstract**

The present invention relates to a dosage form for transmucosal administration comprising a therapeutically effective amount of aliskiren or a pharmaceutically acceptable salt thereof, nd an excipient for transmucosal delivery, wherein the active ingredient is present in an amount of 0.001 to 80% by weight based on the total weight of the dosage form.

## Description

The present invention relates to a dosage form for transmucosal administration of aliskiren, or a pharmaceutically acceptable salt thereof, comprising a therapeutically effective amount of aliskiren, or a pharmaceutically acceptable salt thereof and an excipient for transmucosal delivery. In particular, the present invention provides such galenic formulations comprising aliskiren, preferably, a hemi-fumarate salt thereof, alone or in combination with another active agent. The present invention also relates to their use as medicaments.

In the following the term "aliskiren", if not defined specifically, is to be understood both as the free base and as a salt thereof, especially a pharmaceutically acceptable salt thereof, most preferably a hemi-fumarate thereof.

Renin released from the kidneys cleaves angiotensinogen in the circulation to form the decapeptide angiotensin I. This is in turn cleaved by angiotensin converting enzyme in the lungs, kidneys and other organs to form the octapeptide angiotensin II. The octapeptide increases blood pressure both directly by arterial vasoconstriction and indirectly by liberating from the adrenal glands the sodium-ion-retaining hormone aldosterone, accompanied by an increase in extracellular fluid volume. Inhibitors of the enzymatic activity of renin bring about a reduction in the formation of angiotensin I. As a result a smaller amount of angiotensin II is produced. The reduced concentration of that active peptide hormone is the direct cause of, e.g., the antihypertensive effect of renin inhibitors. Accordingly, renin inhibitors, or salts thereof, may be employed, e.g., as antihypertensives or for treating congestive heart failure.

The renin inhibitor, aliskiren, in particular, a hemi-fumarate thereof, is known to be effective in the treatment of reducing blood pressure irrespective of age, sex or race and is also well tolerated. Aliskiren in form of the free base is represented by the following formula 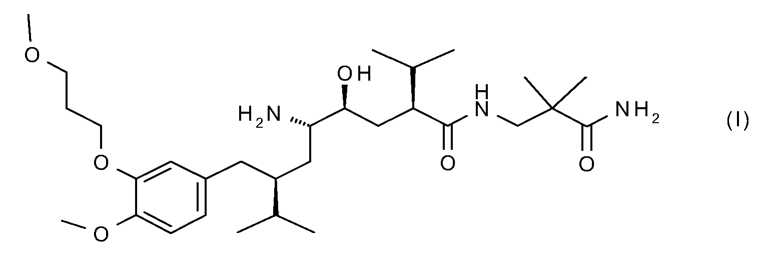 and chemically defined as 2(*S*),4(*S*),5(*S*),7(*S*)-*N*-(3-amino-2,2-dimethyl-3-oxopropyl)-2,7-di(1-methylethyl)-4-hydroxy-5-amino-8-[4-methoxy-3-(3-methoxy-propoxy)phenyl]-octanamide. As described above, most preferred is the hemi-fumarate salt thereof which is specifically disclosed in EP 678503 A as Example 83.

The oral administration of such pharmaceutical agents as tablets or capsules has certain advantages over parenteral administration such as i.v. or i.m. Diseases requiring treatment with painful injectable formulations are considered to be more serious than those conditions which can be treated with oral dosage forms. However, the major advantage with oral formulations is held to be their suitability for self administration whereas parenteral formulations have to be administered in most cases by a physician or paramedical personnel.

However, aliskiren is difficult to formulate and heretofore it is not trivial to prepare oral formulations in the form of tablets in a reliable and robust way. In a galenic formulation comprising aliskiren, or a pharmaceutically acceptable salt thereof, a high amount is normally needed of the drug substance (DS) with properties that make the formulation of tablets difficult.

When using the oral administration route, the bioavailability of the therapeutic agent may be reduced by the action of so-called "efflux pump" proteins which actively eject foreign substances from the cell to give rise, for example, to the multidrug resistance effect. These drug efflux proteins principally comprise MDR (multidrug resistance protein) and MRP (multidrug resistance associated protein) type transporters. Some of the best studied efflux proteins include P-glycoprotein (Pgp or MDR1) and MRP2. A method of improving the bioavailability of a renin inhibitor, such as aliskiren, by co-administering with an efflux protein inhibitor has been described in WO2006/013094.

Despite the advantages imparted by the formulations known and described to date, there is an increasing need for formulations that are easy to prepare and to handle and which would improve the bioavailability of aliskiren and, thus, render the therapy with aliskiren less expensive.

The present invention relates to a dosage form for transmucosal administration of aliskiren, or a pharmaceutically acceptable salt thereof, comprising a therapeutically effective amount of aliskiren, or a pharmaceutically acceptable salt thereof and an excipient for transmucosal delivery, wherein the active ingredient is present in an amount of 0.001 to 80% by weight based on the total weight of the dosage form.

Surprisingly, it was found that the bioavailability of aliskiren may be improved by changing the absorption site from oral to transmucosal, such as buccal, nasal, ocular or vaginal, absorption sites. Unlike the oral administration of drugs, this route does not have the disadvantages such as hepatic first pass metabolism and enzymatic degradation within the Gl tract, that prohibit or hinder the oral administration of certain classes of drugs. Transmucosal routes of drug delivery (i.e., the mucosal linings of the nasal, rectal, vaginal, ocular, and oral cavity) have shown to offer distinct advantages over peroral administration for systemic drug delivery for aliskiren. These advantages include possible bypass of first pass effect, avoidance of presystemic elimination within the GI tract and a better enzymatic flora for drug absorption.

In a preferred embodiment, the dosage form is a delivery system for transmucosal delivery using the oral mucosal cavity and buccal delivery. Within the oral mucosal cavity, delivery of drugs is classified into three categories: (i) sublingual delivery, which is systemic delivery of drugs through the mucosal membranes lining the floor of the mouth, (ii) buccal delivery, which is drug administration through the mucosal membranes lining the cheeks (buccal mucosa), and (iii) local delivery, which is drug delivery into the oral cavity. Of these, buccal and sublingual delivery is the most preferred .

In another preferred embodiment, the dosage form is a delivery system for transmucosal delivery using the nasal cavity (nasal delivery).

Preferred examples for delivery by the transmucosal route are sprays, lozenges, capsules, such as soft bite capsules, tablets, such as rapidly disintegrating tablets, in particular lyophilized disintegrating tablets, effervescent oral dosage forms, chewing gums, such as natural gums, thin films, patches, such as bioerodible patches, powders or drops.

For buccal delivery, sprays, thin films, patches, rapidly disintegrating and effervescent oral dosages forms are particularly preferred. For nasal delivery, spays, powders and drops are particularly preferred.

### Sprays

Examples of sprays suitable for the present invention are disclosed, e.g. WO05/032520, WO05/030167, WO05/032517 and WO05/032518. Exemplary sprays may be aerosol sprays or pump sprays. When using a spray, the mucosal membranes are typically coated with fine droplets of spray containing the active compound.

A spray may preferably contain aliskiren in an amount of 0.001 to 60%, more preferably 0.01 to 50 %, most preferably 0.05 to 40%, of the total composition.

Dependent on the type of spray, a solvent is present as an excipient. The solvent can be a non-polar or polar solvent or a mixture of these solvent. The solvent is typically present in an amount of 10 to 99.99%, more preferably 20 to 99.8%, most preferably 30 to 99 %, of total composition. If the spray is a propellant-free spray, such as a pump spray, then the solvent may make up the remaining amount present in addition to aliskiren.

The non-polar solvent is a non-polar hydrocarbon, preferably a C' e hydrocarbon of a linear or branched configuration, fatty acid esters, and triglycerides, such as miglyol. The solvent must dissolve the active compound and be miscible with the propellant, i.e., solvent and propellant must form a single phase at a temperature of 0-40°C a pressure range of between 1-3 atm.

Suitable non-polar solvents include non-polar hydrocarbons, such as C₇ to C₁₈ linear or branched hydrocarbons, fatty acid esters, such as C₂ to C₂₄ fatty acid C₂-C₆ esters, C₂ to C₆ alkanoyl esters, and the triglycerides of the corresponding acids, such as miglyol. As polar solvents there may be used low molecular weight polyethyleneglycols (PEG) of 400-1000 Mw (preferably 400- 600), low molecular weight (C₂-C₈) mono and polyols and alcohols of C₇-C₁₈ linear or branch chain hydrocarbons, glycerin may also be present and water may also be used in the sprays. The solvent must dissolve the active compound. If a propellant is present, the solvent must be miscible with the propellant, i.e., solvent and propellant must form a single phase at a temperature of 0-40°C a pressure range of between 1-3 atm.

The spray may contain further excipients, e.g. an aerosol spray may contain a propellant. The propellant may be present preferably in an amount of 5 to 80%, more preferably 10 to 70 %, of the total composition.

The propellant is a non-Freon material, preferably a C₃ to C₈ hydrocarbon of a linear or branched configuration. The propellant should be substantially non-aqueous. The propellant produces a pressure in the aerosol container such that under expected normal; usage it will produce sufficient pressure to expel the solvent from the container when the valve is activated but not excessive pressure such as to damage the container or valve seals.

As propellants for the non polar sprays, propane, N-butane, iso- butane, N pentane, isopentane, and neo-pentane, and mixtures thereof may be used. It is permissible for the propellant to have a water content of no more than 0. 2%, typically 0.1-0.2%. All percentages herein are by weight unless otherwise indicated. It is also preferable that the propellant be synthetically produced to minimize the presence of contaminants which are harmful to the active compounds. These contaminants include oxidizing agents, reducing agents, Lewis acids or bases, and water. The concentration of each of these should be less than 0.1 %, except that water may be as high as 0.2%.

In order to mask any unpleasant taste, a flavoring agent, if necessary or desired may be added. The flavoring agent, if present, is preferably employed in an amount of 0.05 to 15 %, more preferably 0.1 to 10%, most preferably 0.5 to 8%, of the total composition.

The preferred flavoring agents are synthetic or natural oil of peppermint, oil of spearmint, citrus oil, fruit flavors, sweeteners (sugars, aspartame, saccharin, etc.), and combinations thereof. The flavoring agent may also include a taste mask. The term "taste mask" as used herein means an agent that can hide or minimize an undesirable flavor such as a bitter or sour flavor. A representative taste mask is a combination of vanillin, ethyl vanillin, malted, iso-amyl acetate, ethyl oxyhydrate, anisic aldehyde, and propylene glycol (commercially available as "PFC 9885 Bitter Mask" from Pharmaccutical Flavor Clinic of Camden, NJ). A taste mask in combination with a flavoring agent is particularly advantageous when the active compound is an alkaloid since alkaloids often have a bitter taste.

### Soft bite capsules

Examples of soft bite capsules suitable for the present invention are disclosed, e.g. WO99/016417.. When using a soft bite capsule, the mucosal membranes are typically coated with a solution or paste of the capsule containing the active compound.

The composition of the soft bite capsules with respect to the amounts and types of excipients is similar to the above-described spray. In order to obtain the desired consistency, the capsule should contain not more than 10% water. When the capsule fill is a paste, other liquid components may be used instead of the above low molecular weight solvents. These include soya oil, corn oil, other vegetable oils.

### Rapidly disintegrating tablets

Examples of rapidly disintegrating tablets suitable for the present invention are disclosed, e.g. in US patents US5976577, US6413549, US6680071 and US6509040.

The term "rapidly disintegrating" as used herein means that the solid dosage form will disintegrate in water at 37.degree. C. in 60 seconds or less. The forms usually disintegrate in about 5-20 seconds, more usually 5 to 10 seconds or less, when tested by the following procedure which is analogous to the Disintegration Test for Tablets, B.P. 1973 which is described in British patent number 1548022..

A rapidly disintegrating tablet may preferably contain aliskiren in an amount of 0.1 to 98%, more preferably 1 to 90 %, most preferably 10 to 60%, of the total composition.

As a further excipient, the rapidly disintegrating tablet may contain a carrier material for aliskiren. The carrier material allows to form structure which will lead to the rapid disintegration of the table. The carrier material which forms a network or matrix containing aliskiren may be any water-soluble or water-dispersible material that is pharmaceutically acceptable, inert to the pharmaceutically active substance and which is capable of forming a rapidly disintegrating network, i.e. disintegrates within 10 seconds or less in the mouth. The preferred carrier material for use in the present invention is gelatin, usually pharmaceutical grade gelatin. Other substances may be used as the carrier material are, for example, hydrolyzed dextrose, dextran, dextrin, maltodextrin, alginates, hydroxyethyl cellulose, sodium carboxymethyl cellulose, microcrystalline cellulose, corn-syrup solids, pectin, carrageenan, agar, chitosan, locust bean gum, xanthan gum, guar gum, acacia gum, tragacanth, conjac flower, rice flower, wheat gluten, sodium starch glycolate, soy fiber protein, potato protein, papain, horseradish peroxidase, glycine and mannitol. Most preferably, gelatin is used.

The tablets may contain a coating on the aliskiren particles. Generally, the coating on the particles is a polymer or lipid material and serves to prevent loss of the pharmaceutical agent during processing, as well as delaying release of the pharmaceutically active substance beyond the point of disintegration of the form in the mouth. Any suitable polymer or lipid or combination can be used as the coating material. Examples of suitable polymers include cellulose and derivatives such as ethylcellulose, hydroxyethylcellulose, hydroxypropylmethylcellulose, hydroxypropylcellulose, methylcellulose, cellulose acetate, cellulose acetate phthalate, hydroxypropylmethylcellulosephthalate, acrylic derivatives, such as polymethacrylates, polyglycolic--polylactic acid, polyvinylalcohol, gelatin, collagen and polyethyleneglycol. Examples of suitable lipid materials include waxes such as beeswax and lanolin, stearic acid and derivatives such as glycerol esters, fixed oils, fats, phospholipids, and glycolipids

An agent may be added to the suspension when forming the rapidly disintegrating tablet which gives increased structural integrity to the matrix. The structure-forming agent is typically a polyhydric alcohol, for example mannitol or sorbitol. The structure-forming agent is normally added to the suspension in an amount of about 1-5% by weight, for example about 2-4% by weight.

Information on the process of preparing such tablets can be taken from the US patents mentioned above. Preferred is a lyophilized rapidly disintegrating tablet.

### Effervescent tablets

Examples of effervescent tablets suitable for the present invention are disclosed, e.g. in WO00/57858.

An effervescent tablet may preferably contain aliskiren in an amount of 0.1 to 98%, more preferably 1 to 90 %, most preferably 10 to 60%, of the total composition.

The effervescent tablets should include as an excipient an effervescent. Typically, the effervescent is present in an amount o effective to aid in penetration of the drug across the oral mucosa. Preferably, the effervescent is provided in an amount of between about 5% and about 95% by weight, based on the weight of the finished tablet, and more preferably in an amount of between about 30% and about 80% by weight. It is particularly preferred that sufficient effervescent material be provided such that the evolved gas is more than about 5 cm³ but less than about 30 cm³, upon exposure of the tablet to an aqueous environment.

The term "effervescent "includes compounds which evolve gas. The preferred effervescent agents evolve gas by means of a chemical reaction which takes place upon exposure of the effervescent agent (an effervescent couple) to water and/or to saliva in the mouth. This reaction is most often the result of the reaction of a soluble acid source and a source of carbon dioxide such as an alkaline carbonate or bicarbonate. The reaction of these two general compounds produces carbon dioxide gas upon contact with water or saliva. Such water- activated materials must be kept in a generally anhydrous state and with little or no absorbed moisture or in a stable hydrated form, since exposure to water will prematurely disintegrate the tablet. The acid sources may be any which are safe for human consumption and may generally include food acids, acid and hydrite antacids such as, for example: citric, tartaric, amalic, fumeric, adipic, and succinics. Carbonate sources include dry solid carbonate and bicarbonate salt such as, preferably, sodium bicarbonate, sodium carbonate, potassium bicarbonate and potassium carbonate, magnesium carbonate and the like. Reactants which evolve oxygen or other gasses and which are safe for human consumption are also included.

The effervescent agent(s) used in the present invention is not always based upon a reaction which forms carbon dioxide.

Where the effervescent includes two mutually reactive components, such as an acid source and a carbonate source, it is preferred that both components react completely. Therefore, an equivalent ratio of components which provides for equal equivalents is preferred. For example, if the acid used is diprotic, then either twice the amount of a mono-reactive carbonate base, or an equal amount of a di-reactive base should be used for complete neutralization to be realized. However in other embodiments of the present invention, the amount of either acid or carbonate source may exceed the amount of the other component. This may be useful to enhance taste and/or performance of a tablet containing an overage of either component. In this case, it is acceptable that the additional amount of either component may remain unreacted.

The present dosage forms may also include in amounts additional to that required for effervescence a pH adjusting substance. For drugs that are weakly acidic or weakly basic, the pH of the aqueous environment can influence the relative concentrations of the ionized and unionized forms of the drug present in solution according to the Henderson-Hasselbach equation. The pH solutions in which an effervescent couple has dissolved is slightly acidic due to the evolution of carbon dioxide. The pH of the local environment, e.g., saliva in immediate contact with the tablet and any drug that may have dissolved from it, may be adjusted by incorporating in the tablet a pH adjusting substances which permit the relative portions of the ionized and unionized forms of the drug to be controlled. In this way, the present dosage forms can be optimized for each specific drug. If the unionized drug is known or suspected to be absorbed through the cell membrane (transcellular absorption) it would be preferable to alter the pH of the local environment (within the limits tolerable to the subject) to a level that favors the unionized form of the drug.

Suitable pH adjusting substance for use in the present invention include any weak acid or weak base in amounts additional to that required for the effervescence or, preferably, any buffer system that is not harmful to the oral mucosa. Suitable pH adjusting substance for use in the present invention include, but are not limited to, any of the acids or bases previously mentioned as effervescent compounds, disodium hydrogen phosphate, sodium dihydrogen phosphate and the equivalent potassium salt.

In addition to the effervescence-producing agents, a dosage form according to the present invention may also include suitable non-effervescent disintegration agents. Non-limiting examples of non-effervescent disintegration agents include: microcrystalline, cellulose, croscarmelose sodium, - WO 00/57858 PCT/US00/075677 crospovidone, starches, corn starch, potato starch and modified starches thereof, sweeteners, clays, such as bentonite, alginates, gums such as agar, guar, locust bean, karaya, pecitin and tragacanth. Disintegrants may comprise up to about 20 weight percent and preferably between about 2 and about 10% of the total weight of the composition.

In addition to the particles in accordance with the present invention, the dosage forms may also include glidants, lubricants, binders ' sweeteners, flavoring and coloring components. Any conventional sweetener or flavoring component may be used. Combinations of sweeteners, flavoring components, or sweeteners and flavoring components may likewise be used.

Examples of binders which can be used include acacia, tragacanth, gelatin, starch, cellulose materials such as methyl cellulose and sodium carboxy methyl cellulose, alginic acids and salts thereof, magnesium aluminum silicate, polyethylene glycol, guar gum, polysaccharide acids, bentonites, sugars, invert sugars and the like. Binders may be used in an amount of up to 60 weight percent and preferably about 10 to about 40 weight percent of the total composition.

Coloring agents may include titanium dioxide, and dyes suitable for food such as those known as F.D.&. dyes and natural coloring agents such as grape skin extract, beet red powder, beta-carotene, annato, carmine, turmeric, paprika, etc. The amount of coloring used may range from about 0.1 to about 3.5 weight percent of the total composition.

Flavors incorporated in the composition may be chosen from synthetic flavor oils and flavoring aromatics and/or natural oils, extracts from plants, leaves, flowers, fruits and so forth and combinations thereof. These may include cinnamon oil, oil of wintergreen, peppermint oils, clove oil, bay oil, anise oil, eucalyptus, thyme oil, cedar leave oil, oil of nutmeg, oil of sage, oil of bitter almonds and cassia oil.

Also useful as flavors are vanilla, citrus oil, including lemon, orange, grape, lime and grapefruit, and fruit essences, including apple, pear, peach, strawberry, raspberry, cherry, plum, pineapple, apricot and so forth. Flavors which have been - WO 00/57858 PCT/US00/07567 8 found to be particularly useful include commercially available orange, grape, cherry and bubble gum flavors and mixtures thereof. The amount of flavoring may depend on a number of factors, including the organoleptic effect desired. Flavors may be present in an amount ranging from about 0.05 to about 3 percent by weight based upon the weight of the composition.

Particularly preferred flavors are the grape and cherry flavors and citrus flavors such as orange.

One aspect of the invention provides an effervescent solid, oral tablet dosage form suitable for sublingual, buccal, and gingival administration. Excipient fillers can be used to facilitate tableting. The filler desirably will also assist in the rapid dissolution of the dosage form in the mouth. Non-limiting examples of suitable fillers include: mannitol, dextrose, lactose, sucrose, and calcium carbonate.

### Thin film and patches

Examples of thin films and patches suitable for the present invention are disclosed, e.g. in US5192802 (bioadhesive gels), US45518721 (denture adhesive pastes), US5,800,832 and 6,159, 498 and US 6585997 (bioerodible films) as well as in Amir H Shojaei et al., J Pharm Pharmaceut Sci (www.ualberta.ca/~csps) 1 (1):15-30, 1998 (comprehensive review on buccal patches, including references cited therein).

A thin film or patch may preferably contain aliskiren in an amount of 0.001 to 50%, more preferably 0.002 to 30 %, most preferably 0.005 to 20%, of the total composition.

Thin films and patches are devices that are applied to mucosal surfaces and provide protection of the application site while delivering pharmaceuticals to treat specific diseases or disorders. The device causes minimum discomfort, is easy to use and provides an effective residence time that can be tailored to deliver therapeutics over different time intervals. In one embodiment, the device comprises a mucoadhesive multi-layered film disc that is water-soluble and bioerodable. In another embodiment, the device comprises a multi-layered film having an adhesive layer and a coated backing layer containing aliskiren in either or both layers. The film may be cut or fabricated into any desired shape, such as a disc, square, oval, parallelepiped, etc., that provides convenience for use in application and/or treatment. The adhesive layer of the device is water soluble and the backing layer is bioerodible.

The adhesive layer preferably comprises a film-forming polymer such as hydroxyethyl cellulose, hydroxypropyl cellulose, hydroxypropylmethyl cellulose, or hydroxyethylmethyl cellulose, alone or in combination, and a bioadhesive polymer such as polyacrylic acid, polyvinyl pyrrolidone, or sodium carboxymethyl cellulose, alone or in combination. The non-adhesive backing layer is preferably a precast film alone or in combination with other layers. The precast film is preferably comprised of hydroxyethyl cellulose, hydroxypropyl cellulose, hydroxyethylmethyl cellulose, hydroxypropylmethyl cellulose, polyvinyl alcohol, polyethylene glycol, polyethylene oxide, ethylene oxide-propylene oxide co-polymers, or other water soluble film-forming polymer, alone or in combination thereof. The precast film may also include plasticizers or other excipients required to enhance the film forming properties of the polymer. The non-adhesive backing layer is further modified to render it water erodible instead of water soluble. For definition purpose, water erodible means a material or substance that does not dissolve in in water or bodily fluids in total, however will disintegrate and completely break apart upon exposure to water or bodily fluids. This is accomplished by coating the backing layer film with a more hydrophobic polymer selected from a group of Eudragit® and/or ethyl cellulose and methyl cellulose polymers that are approved by the FDA for use in pharmaceutical applications. Other hydrophobic polymers known to those skilled in the art may also be used. The type and amount of hydrophobic polymer used will provide a wide and controlled range of Residence Times for the layered disk device. In addition, the modified, precast backing layer eliminates the need to use a rigid support material such as a polyethylene film or other non-porous material as the casting surface on which both the adhesive layer and backing layer are produced. This casting surface is no longer an integral component of the layered device, which from a safety and production point of view, is extremely desirable.

The mucoadhesive erodible multi layered device comprises preferably a first water soluble adhesive layer to be placed in contact with a mucosal surface and second water erodible non- adhesive backing layer that controls residence time of the device. Residence time, the time for which device in placed on the target mucosal surface will remain substantially intact). The first layer preferably comprises at least one water soluble film forming element in combination with at least one mucoadhesive polymer. The second water erodible non adhesive backing layer preferably comprises a precast film containing at least one of hydroxypropyl methylcellulose, hydroxyethyl cellulose, hydroxypropyl cellulose, polyvinyl alcohol, polyethylene glycol, polyethylene oxide, and ethylene oxide-propylene oxide copolymer. This layer is coated with at least one hydrophobic polymer alone or in combination with at least one hydrophilic polymer, such that the layer is bioerodible.

This device may be used by itself or may be used with aliskiren incorporated therein. This device may be used for the protection of a mucosal site and/or the administraton of aliskiren locally, regionally or systemically. In a preferred embodiment, the first water-soluble adhesive layer comprises at least one water-soluble film-forming polymer selected from the group consisting of hydroxyethyl cellulose, hydroxypropyl cellulose, hydroxypropyl methylcellulose, and hydroxyethyl methylcellulose, in combination with at least one mucoadhesive polymer selected from the group consisting of polyacrylic acid, polyvinyl pyrrolidone, and sodium carboxymethyl cellulose. The second water erodible non-adhesive backing layer may act as a casing and support surface on which the adhesive layer is prepared. This second layer preferably comprises a premade film of hydroxypropyl methylcullulose in combination with a coating consisting of at least one hydrophobic polymer selected from the family of Eudragit polymers, ethyl cellulose and methylcellulose alone or in combination with at least hydrophilic polymer selected from the group consisting of polyvinyl pyrrolidone, hydroxypropylmethylcellulose, hydroxyethylmethylcellulose, hydroxypropylcellulose and polyvinylalcohol.

In certain preferred embodiments the mucoadhesive bioerodible multilayered device o has a second water erodible non- adhesive backing layer that comprises a pre-made film of hydroxypropyl methylcullulose and a coating of a hydrophobic and hydrophilic polymer mixture at a ratio of 0.5:1 to 18:1. A more preferred ratio is 1:0 to 10:1.

The non-adhesive backing layer of the device of the present invention preferably comprises a precast film of hydroxypropyl methylcellulose with a coating mixture of hydrophobic and hydrophilic polymers at a ratio of 1:0 to 10:1. This coating contains at least one of propylene glycol, polyethylene glycol or glycerine as a plasticizer to improve flexibility. A preferred non-adhesive backing layer of the device of the present invention comprises a premade film of hydroxypropyl methylcellulose and a coating mixture of hydrophobic and hydrophilic polymers at a ratio of 1:0 to 10:1. A preferred coating mixture contains at least one of hyaluronic acid and an alpha hydroxyl acid as a humectant to improve softness or feel. A preferred humectant is glycolic acid.

In one particularly preferred embodiment, the mucoadhesive erodible multi layered device has an non-adhesive backing layer that comprises a precast film of hydroxypopyl methylcellulose and a coating mixture of hydrophobic and hydrophilic polymers at a ratio of 1:0 to 10:1. A preferred coating mixture contains titanium dioxide, zinc oxide or zirconium silicate as an opacifier and one or less FD& Red, Yellow, Green or Blue as a coloring agent to help distinguish the backing layer from the mucoadhesive layer. In one embodiment, the backing layer of the present device comprises a premade film of hydroxypropyl methylcellulose, a coating comprising a mixture of hydrophobic and hydrophilic polymers at a ratio of 1:0 to 10:1, a plasticizer and a coloring agent or an opacifier whose combined total is less than about 4% by weight of the device.

In a very important embodiment of the present invention, the mucoadhesive, erodible multi-layered device further comprises aliskiren within said first or second layer. Aliskiren may be incorporated within the first or second layers of the device of the present invention. These layers may each independently comprise flavoring agent to mask the taste of any pharmecutical agent or simply to improve patient compliance.

### Permeation/absorption enhancers

The delivery systems in accordance with the present invention may be used in conjunction with permeation/absorption enhancers known in the art. Suitable examples include
- Anionic surfactants (e.g. sodium lauryl sulfate, sodium laureate)
- Cationic surfactants (e.g. cetylpyridinium chloride)
- Nonionic surfactants (e.g. Polysorbate-80)
- Bile salts (e.g. Sodium glycodeoxycholate, Sodium glycocholate, Sodium taurodeoxycholate, Sodium taurocholate)
- Polysaccharides (e.g. Chitosan)
- Synthetic polymers (e.g. Carbopol, Carbomer)
- Fatty acids (e.g. Oleic acid, Caprylic acid)
- Chelators (e.g. EDTA = Ethylenediaminetetraacetic acid, Sodium citrate)
- Cyclodextrins: α, β, γ cyclodextrins

For a general review and insights on mechanism of action of absorption (permeation) enhancers for buccal application such as increasing the fluidity of the cell membrane, extracting inter/intracellular lipids, altering cellular proteins or altering surface mucin it is referred to SENEL S, HINCAL AA: Drug permeation enhancement via the buccal route: possibilities and limitations. J. Control. Rel. (2001) 72:133-144.

The most common absorption enhancers are fatty acids, bile salts and surfactants. Detail of their use and amounts are provided in MORISHITA M, BARICHELLO JM, TAKAYAMA K, CHIBA Y, TOKIWA S, NAGAI T: Pluronic F-127 gels incorporating highly purified unsaturated fatty acids for buccal delivery of insulin. Int. J. Pharm. (2001) 212:289-293. TSUTSUMI K, OBATA Y, NAGAI T, LOFTSSON T, TAKAYAMA K: Buccal absorption of ergotamine tartrate using the bioadhesive tablet system in guinea pigs. Int.J. Pharm. (2002) 238:161-170.

Bile salts have been used extensively as penetration enhancers, and are believed to act by the extraction of lipids or proteins from the cell wall, membrane fluidisation and reverse membrane micellation without causing major damage to the buccal mucosa. For more detail, reference is made to VEUILLEZ F, KALIA YN, JACQUES Y, DESHUSSES J, BURI P: Factors and strategies for improving buccal absorption of peptides. Eur. J. Pharm. Biopharm. (2001) 51:93-109.

A range of other materials has also been reported to have absorption-enhancing effects. For example, solutions/gels of chitosan were found to promote the transport of mannitol and fluorescentlabelled dextrans across a tissue culture model of the buccal epithelium. Reference is made to PORTERO A, REMUNAN-LOPEZ C, NIELSEN, HM: The potential of chitosan in enhancing peptide and protein absorption across the TR146 cell culture model-an in vitro model of the buccal epithelium. Pharm. Res. (2002) 19:169-174.

Glyceryl monooleates were reported to enhance peptide absorption by a co-transport mechanism, see for more detail LEE J, KELLAWAY W: Buccal permeation of [D-Ala2 D-Leu5]enkephalin from liquid crystalline phases of glyceryl monooloeate. Int. J. Pharm. (2000) 195:3538.

The lipophilic skin-penetration enhancers octisalate, padimate (both used in sun screens) and laurocapram on the buccal absorption of various drugs *in vitro* have been described in e.g. NICLAZZO JA, REED BL, FINNIN BC: Modification of buccal delivery following pretreatment with skin penetration enhancers. J. Pharm. Sci. (2004) 93(8):2054-2063**,** and are equally applicable.

The inhibition of enzymes that may degrade biopharmaceutical drugs can also enhance absorption and materials such as aprotinin and puromycin have been added to reduce peptide degradation. Reference is made to YAMAMOTO A, HAYAKAWA E, LEE VH: Insulin and proinsulin proteolysis in mucosal homogenates of the albino rabbit: implications in peptide drug delivery rom non-oral routes. Life Sci. (1990) 47:2465-2474. and TAVAKOLI-SABERI MR, WILLIAMS A, AUDUS KL: Aminopeptidase activity in human buccal epithelium and primary cultures of hamster buccal epithelium. Pharm. Res. (1991) 6:S197.

The above described enhancers are suitable for the purpose of the present invention.

The dosage form in accordance with the present invention contains aliskiren in a therapeutically effective amount, preferably as mentioned above for the individual dosage forms. The terms "effective amount" or "therapeutically effective amount" refers to the amount of the active ingredient or agent which halts or reduces the progress of the condition being treated or which otherwise completely or partly cures or acts palliatively on the condition.

Aliskiren, or a pharmaceutically acceptable salt thereof, can, e.g., be prepared in a manner known *per se,* especially as described in EP 678503 A, e.g., in Example 83.

One or more of the excipients mentioned above for the individual delivery systems can be selected and used by a person skilled in the art having regard to the particular desired properties of the dosage form for transmucosal administration by routine experimentation and without any undue burden.

The dosage form for transmucosal administration of the present invention are useful for lowering the blood pressure, either systolic or diastolic or both. The conditions for which the instant invention is useful include, without limitation, hypertension (whether of the malignant, essential, reno-vascular, diabetic, isolated systolic, or other secondary type), congestive heart failure, angina (whether stable or unstable), myocardial infarction, artherosclerosis, diabetic nephropathy, diabetic cardiac myopathy, renal insufficiency, peripheral vascular disease, left ventricular hypertrophy, cognitive dysfunction (such as Alzheimer's) and stroke, headache and chronic heart failure.

The present invention likewise relates to a method of treating hypertension (whether of the malignant, essential, reno-vascular, diabetic, isolated systolic, or other secondary type), congestive heart failure, angina (whether stable or unstable), myocardial infarction, artherosclerosis, diabetic nephropathy, diabetic cardiac myopathy, renal insufficiency, peripheral vascular disease, left ventricular hypertrophy, cognitive dysfunction, e.g., Alzheimer's, stroke, headache and chronic heart failure comprising administering to an animal, including human patient, in need of such treatment a therapeutically effective amount of the dosage form for transmucosal administration according to the present invention.

The present invention likewise relates to the use of a s dosage form for transmucosal administration according to the present invention for the manufacture of a medicament for the treatment of hypertension (whether of the malignant, essential, reno-vascular, diabetic, isolated systolic, or other secondary type), congestive heart failure, angina (whether stable or unstable), myocardial infarction, artherosclerosis, diabetic nephropathy, diabetic cardiac myopathy, renal insufficiency, peripheral vascular disease, left ventricular hypertrophy, cognitive dysfunction, e.g., Alzheimer's, stroke, headache and chronic heart failure.

The present invention likewise relates to a pharmaceutical composition for the treatment of hypertension (whether of the malignant, essential, reno-vascular, diabetic, isolated systolic, or other secondary type), congestive heart failure, angina (whether stable or unstable), myocardial infarction, artherosclerosis, diabetic nephropathy, diabetic cardiac myopathy, renal insufficiency, peripheral vascular disease, left ventricular hypertrophy, cognitive dysfunction, e.g., Alzheimer's, stroke, headache and chronic heart failure, comprising a s dosage form for transmucosal administration according to the present invention.

Ultimately, the exact dose of the active agent and the particular formulation to be administered depend on a number of factors, e.g., the condition to be treated, the desired duration of the treatment and the rate of release of the active agent. For example, the amount of the active agent required and the release rate thereof may be determined on the basis of known *in vitro* or *in vivo* techniques, determining how long a particular active agent concentration in the blood plasma remains at an acceptable level for a therapeutic effect.

The above description fully discloses the invention including preferred embodiments thereof. Modifications and improvements of the embodiments specifically disclosed herein are within the scope of the following claims. Without further elaboration, it is believed that one skilled in the art can, using the preceding description, utilize the present invention to its fullest extent.

Therefore, the Examples herein are to be construed as merely illustrative and not a limitation of the scope of the present invention in any way.

### Example 1:

### Buccal spray containing Aliskiren hemifumarate

### (amounts in Weight %)

| | **Amounts** | **preferred amount** | **most preferred amount** |
|---|---|---|---|
| aliskiren hemifumarate | 0.001-0.5 | 0.005-0.250 0. | 01-0. 10 |
| acetic acid | 1-10 | 2-8 | 4-6 |
| sodium acetate | 1-10 | 2-8 | 4-6 |
| sodium chloride | 3-30 | 5- 25 | 15-20 |
| flavors | 0.1-5 | 0.5-4 2-3 | |
| ethanol | 5-30 | 7.5-20 | 9.5-15 |
| water | 15-95 | 35-90 | 65-85 |

### Example 2:

### Buccal spray containing Aliskiren hemifumarate

### (amounts in Weight %)

| | **Amounts** | **preferred amount** | **most preferred amount** |
|---|---|---|---|
| aliskiren hemifumarate | 0.1-10 | 0. 2-7 | 0.25-5 |
| water | 50-95 | 60-80 | 65-75 |
| ethanol | 5-20 | 7.5-15 | 9.5-12.5 |
| polyethylene glycol | 5-20 | 7.5-15 | 9.5-12.5 |
| flavors | 1-10 | 2-8 | 3-6 |

### Example 3:

### Soft bite capsule containing Aliskiren hemifumarate

### (amounts in Weight %)

| | **Amounts** | **preferred amount** | **most preferred amount** |
|---|---|---|---|
| aliskiren hemifumarate | 30-85 | 40-75 | 45- 55 |
| soya oil | 7.5-50 | 10-40 | 12.5-35 |
| soya lecithin | 0.001-1.0 | 0.005-0.5 | 0.01-0.1 |
| Soya fats | 7.5-50 | 10-40 | 12.5-35 |
| flavors | 1-10 | 2-8 | 3-6 |

### Example 4:

### Soft bite capsule containing Aliskiren hemifumarate

### (amounts in Weight %)

| | **Amounts** | **preferred amount** | **most preferred amount** |
|---|---|---|---|
| aliskiren hemifumarate | 0.01-5 | 0.05-3.5 | 0.075-1.75 |
| polyethylene glycol | 25-70 | 30-60 | 35-50 |
| glycerin | 25-70 | 30-60 | 35-50 |
| flavors | 0.1-10 | 1-8 | 3-6 |

### Example 5:

### Mucoadhesive patch containing Aliskiren hemifumarate

A 300 gram batch of mucoadhesive coating solution was prepared using 268.2 grams of deionized water, 5.40 grams of hydroxyethyl cellulose, Natrosol 250 L NF (B F Goodrich), 4.0 g aliskiren hemifumatate, 7.81 grams Noveon AA1, Polycarbophil (B F Goodrich), 13. 50 grams sodium carboxymethyl cellulose, 7LF PH (B F Goodrich), 0.96 grams sodium benzoate, NF(Spectrum Chemicals), and 0.95 grams propylene glycol, USP(Spectrum Chemicals). A Lighnin® mixer with an A-1 00 propeller was used to effectively homogenize this viscous mucoadhesive coating suspension at a speed of 1000 rpm. The resulting percentage of film forming polymer was 1.8% and the mucoadhesive polymers was 7.1%. This adhesive coating suspension was used as shown below.

A hydrophobic coating solution was prepared using stock solutions of both polyvinylpyrrolidone, 16% w/w of PVP, USP, one million molecular weight(BASF), dissolved in ethanol, USP, 190 proof(Spectrum Chemicals), and Eudragit® RS-100 NF(quaternary ammonium acrylate/methacrylate co-polymers) (Rohm GmbH), 48% w/w of polymer dissolved in ethanol, USP, 190 proof. Aliquots of both stock solutions were combined using a lightning mixer to create a coating solution of: twenty grams of pvp solution plus 23.33 grams of Eudragit® solution produced a mixed coating solution ratio of 3.5:1 (Eudragit®: pvp ) Multi-layered films were prepared using the hydrophobic coatings solution outlined in above with the mucoadhesive coating suspension detailed above. First, a piece of hydroxypropyl methyl cellulose precast film(Watson Polymer Films), 0.004 inches thick was cut approximately 18 inches×11.5 inches and placed in the paper and foil holder of a Werner Mathis AG Lab Coater, type LTF. The doctor blade setting was adjusted to 0.15 mm. and each solution from example 3 was applied to individual precast pieces of the backing film. The films were then automatically dried in the oven portion of the lab coater, and a smooth, integral layer of deposited hydrophobic/water soluble polymer resulted. Each coated film was removed and put back into the frame with the uncoated side of the backing layer facing up. The adhesive coating suspension from example 1. was then used to coat each of the coated backing layer samples, using a 1.2 mm. setting on the doctor blade. The films were dried as before, and a second coating and drying step using the adhesive layer was conducted.

### Example 6:

### Lyophilized rapidly disintegrating tablet containing Aliskiren hemifumarate

### (amounts in Weight %)

| | |
|---|---|
| Purified water | 85.10 |
| Gelatin | 2.80 |
| Mannitol | 2.10 |
| Aliskiren hemifumarate | 10.00 |
| FDC Blue No. 2 | 0.0025 |

The gelatin and mannitol were added to the water and heated to 40. degree. C. to dissolve before allowing to cool to 23.degree. C. The mix was gradually added to the alikiren hemifumarate powder with manual mixing until a fluid suspension was formed. The remainder of the solution was then added. Stirring was maintained in a thermostated water bath at 23.degree. C. A 20 ml sample was transferred to a 20 ml glass vial and allowed to stand. A sample was also taken which was then frozen rapidly at -80.degree. C. Freeze drying was then performed using a standard cycle.

### Example 7:

### Effervescent tablet containing Aliskiren hemifumarate

### (amounts in Weight %)

| | |
|---|---|
| aliskiren hemifumarate | 5.00 |
| Sodium Bicarbonate | 15.52 |
| Citric Acid, Anhydrous | 11.08 |
| Sodium Bicarbonate | 45.78 |
| HPMC K4M Prem | 5.00 |
| Dicalcium phosphate | 5.00 |
| dihydrate Mannitol | 11.67 |
| Magnesium Stearate | 0.95 |

These examples exhibited a better bioavailability than comparable oral formulations and the amount of drug substance could be reduced effectively without any adverse effects such as irritation of the mucosa.

## Claims

1. A dosage form for transmucosal administration of aliskiren, or a pharmaceutically acceptable salt thereof, comprising a therapeutically effective amount of aliskiren, or a pharmaceutically acceptable salt thereof and an excipient for transmucosal delivery, wherein the active ingredient is present in an amount of 0.001 to 80% by weight based on the total weight of the dosage form.

2. A dosage form according to Claim 1, wherein the dosage form is a delivery system for buccal delivery.

3. A dosage form according to Claim 1, wherein the dosage form is a delivery system for nasal delivery.

4. A dosage form according to any of the preceding claims, in the form of a buccal or nasal spray.

5. A dosage form according to claim 4, wherein the excipient is a polar or non-polar solvent.

6. A dosage form according to any of claims 1 or 2, in the form of an effervescent oral dosage form.

7. A dosage form according to claim 6, wherein the excipient is an effervescent.

8. A dosage form according to any of claims 1 or 2, in the form of a rapidly disintegrating oral dosage form, including a lyophilized disintegrating tablet.

9. A dosage form according to claim 8, wherein the excipient is a carrier material for a rapidly disintegrating oral dosage form, such as gelatin.

10. A dosage form according to any of claims 1 or 2, in the form of a buccal patch.

11. A dosage form according to claim 10, wherein the excipient is a water- soluble or - insoluble film-forming polymer alone or in combination with at least one mucoadhesive polymer.

12. A dosage form according to any of the preceding claims, comprising a penetration or permeation enhancer.

13. A dosage form according to any of the preceding claims for the treatment of hypertension, congestive heart failure, angina, myocardial infarction, artherosclerosis, diabetic nephropathy, diabetic cardiac myopathy, renal insufficiency, peripheral vascular disease, left ventricular hypertrophy, cognitive dysfunction, stroke, headache and chronic heart failure.

14. A method for the treatment of hypertension, congestive heart failure, angina, myocardial infarction, artherosclerosis, diabetic nephropathy, diabetic cardiac myopathy, renal insufficiency, peripheral vascular disease, left ventricular hypertrophy, cognitive dysfunction, stroke, headache and chronic heart failure which method comprises administering a therapeutically effective amount of a dosage form according to any of claims 1 to12 to a patient in need thereof.

15. Use of a solid oral dosage form according to any of claims 1 to 12 for the manufacture of a medicament for the treatment of hypertension, congestive heart failure, angina, myocardial infarction, artherosclerosis, diabetic nephropathy, diabetic cardiac myopathy, renal insufficiency, peripheral vascular disease, left ventricular hypertrophy, cognitive dysfunction, stroke, headache and chronic heart failure.
